# EUROPEAN PATENT SPECIFICATION

(11) **EP 1 414 859 B1**
(45) Date of publication and mention of the grant of the patent: **14.12.2005**
(21) Application number: 02765138.9
(22) Date of filing: 30.07.2002
(51) Int. Cl.: C07K 16/16, C12N 5/16, G01N 33/574

(54) **ANTI-SSRP-1 MONOCLONAL ANTIBODIES AND HYBRIDOMAS PRODUCING SAID ANTIBODIES**
ANTI-SSRP-1 MONOKLONALE ANTIKÖRPER UND HYBRIDOME ZUR DEREN HERSTELLUNG
ANTICORPS MONOCLONAUX ANTI-SSRP-1 ET HYBRIDOMES PRODUISANT LESDITS ANTICORPS

(30) Priority: 31.07.2001 IT TO20010762
(43) Date of publication of application: 06.05.2004
(73) Proprietor: CONSIGLIO NAZIONALE DELLE RICERCHE, 00185 Roma (IT)
(72) Inventor: GARIGLIO, Marisa, I-10046 Poirino (Torino) (IT); LANDOLFO, Santo, I-10126 Torino (IT)
(74) Representative: Comoglio, Elena
(86) International application number: PCT/IB2002/002955
(87) International publication number: WO 2003/011910

(56) References cited:
- WO-A-93/13222
- GARIGLIO MARISA ET AL: "The high-mobility group protein T160 binds to both linear and cruciform DNA and mediates DNA bending as determined by ring closure." EXPERIMENTAL CELL RESEARCH, vol. 236, no. 2, 1 November 1997 (1997-11-01), pages 472-481, XP002232178 ISSN: 0014-4827 cited in the application
- SANTORO PIERA ET AL: "High prevalence of autoantibodies against the nuclear high mobility group (HMG) protein SSRP1 in sera from patients with systemic lupus erythematosus, but not other rheumatic diseases." JOURNAL OF RHEUMATOLOGY, vol. 29, no. 1, January 2002 (2002-01), pages 90-93, XP008014165 January, 2002 ISSN: 0315-162X

### SEQUENCE LISTING

<110> Consiglio Nazionale delle Ricerche
<120> Anti-SSRP-1 monoclonal antibodies and hybridomas producing said antibodies
<130> PC390PR
<160> 1
<170> PatentIn version 3.1
<210> 1
<211> 408
<212> PRT
<213> Mus musculus
<400> 1
   His Gly Leu Lys Leu Leu Thr Lys Asn Gly His Val Tyr Lys Tyr Asp
   1 5 10 15
   Gly Phe Arg Glu Ser Glu Phe Glu Lys Leu Ser Asp Phe Phe Lys Thr
   20 25 30
   His Tyr Arg Leu Glu Leu Met Glu Lys Asp Leu Cys Val Lys Gly Trp
   35 40 45
   Asn Trp Gly Thr Val Lys Phe Gly Gly Gln Leu Leu Ser Phe Asp Ile
   50 55 60
   Gly Asp Gln Pro Val Phe Glu Ile Pro Leu Ser Asn Val Ser Ser Val
   65 70 75 80
   Pro Gln Ala Arg Ile Glu Val Thr Leu Glu Phe His Gln Asn Asp Asp
   85 90 95
   Pro Glu Val Ser Leu Met Glu Val Arg Phe Tyr Val Pro Pro Thr Gln
   100 105 110
   Glu Asp Gly Val Asp Pro Val Glu Ala Phe Ala Gln Asn Val Leu Ser
   115 120 125
   Lys Ala Asp Val Ile Gln Ala Thr Gly Asp Ala Ile Cys Ile Phe Arg
   130 135 140
   Glu Leu Gln Cys Leu Thr Pro Arg Gly Arg Tyr Asp Ile Arg Ile Tyr
   145 150 155 160
   Pro Thr Phe Leu His Leu His Gly Lys Thr Phe Asp Tyr Lys Ile Pro
   165 170 175
   Tyr Thr Thr Val Leu Arg Leu Phe Leu Leu Pro His Lys Asp Gln Arg
   180 185 190
   Gln Met Phe Phe Val Ile Ser Leu Asp Pro Pro Ile Lys Gln Gly Gln
   195 200 205
   Thr Arg Tyr His Phe Leu Ile Leu Leu Phe Ser Lys Asp Glu Asp Ile
   210 215 220
   Ser Leu Thr Leu Asn Met Asn Glu Glu Glu Val Glu Lys Arg Phe Glu
   225 230 235 240
   Gly Arg Leu Thr Lys Asn Met Ser Gly Ser Leu Tyr Glu Met Val Ser
   245 250 255
   Arg Val Met Lys Ala Leu Val Asn Arg Lys Ile Thr Val Pro Gly Asn
   260 265 270
   Phe Gln Gly His Ser Gly Ala Gln Cys Ile Thr Cys Ser Tyr Lys Ala
   275 280 285
   Ser Ser Gly Leu Leu Tyr Pro Leu Glu Arg Gly Phe Ile Tyr Val His
   290 295 300
   Lys Pro Pro Val His Ile Arg Phe Asp Glu Ile Ser Phe Val Asn Phe
   305 310 315 320
   Ala Arg Gly Thr Thr Thr Thr Arg Ser Phe Asp Phe Glu Ile Glu Thr
   325 330 335
   Lys Gln Gly Thr Gln Tyr Thr Phe Ser Ser Ile Glu Arg Glu Glu Tyr
   340 345 350
   Gly Lys Leu Phe Asp Phe Val Asn Ala Lys Lys Leu Asn Ile Lys Asn
   355 360 365
   Arg Gly Leu Lys Glu Gly Ile Asn Pro Gly Tyr Asp Asp Tyr Ala Asp
   370 375 380
   Ser Asp Glu Asp Gln His Asp Ala Tyr Leu Glu Arg Met Lys Glu Glu
   385 390 395 400
   Gly Lys Ile Arg Glu Glu Asn Ala
   405

## Description

The present invention relates to monoclonal antibodies which react against the protein SSRP-1, to hybridomas producing these antibodies, and to the diagnostic use of these antibodies.

The non-histonic HMG (high-mobility group) chromosomal proteins binding DNA comprise the SSRP ("structure-specific recognition proteins") family which is present in all eucaryotic cells (1, 2). A characteristic common to many HMG proteins, but not to all, is the presence of a DNA binding domain which is known as the HMG box and which differs from other eucaryotic DNA binding domains in several aspects - amongst which is the ability to contact the DNA through the minor groove, the introduction of folds as a result of the bond with the DNA, and a variable specificity for the DNA sequence - which provide a unique DNA-protein recognition mechanism (2).

The classification of 121 HMG domains on the basis of structural alignments has enabled proteins of different kinds to be grouped in a subgroup called the SSRP (structure-specific recognition protein) family (3).

Researchers in the field have only recently started to investigate the various functions of the proteins belonging to this family.

Spencer et al. (4) have used a single hybridization test in yeast to demonstrate that the interaction of the SRF (serum response factor) transcription factor with human protein SSRP-1 dramatically increases its activity in binding to DNA and leads to a synergistic activation of natural and artificial SRF-dependent promoters. It has been found that the homologue of SSRP-1 yeast, known as Pob3, forms a complex with another essential nuclear protein, Cdc68 (5). This complex, which is known as CP complex, is a heterodimer which regulates the transcriptional activation of yeast and the repression of chromatin (6).

More recently, Orphanides et al. have found that the FACT factor (from "facilitates chromatin transcription") a chromatin-specific elongation factor necessary for the transcription of chromatin templates in vitro, comprises SSRP-1 (6).

Moreover, Dyer et al. (7) have demonstrated that SSRP1/PREIIBF acts as a structure-specific transcription factor which brings together proteins bound in positions far apart upstream of the human beta-globin gene.

Finally, Xiang et al. (8) have demonstrated that the mRNA of SSRP1 is expressed specifically in rat foetal kidney and in the carcinoma of renal cells induced by Fenitrilotriacetate, but not in the adult kidney.

The present inventors have demonstrated in previous studies that SSRP1 binds DNA (both cruciform and double-helix linear) without apparent sequence specificity and that it also folds DNA, which is a general characteristic of HMG box proteins (9). Functional studies have revealed that a reduced expression of SSRP1 prejudices the growth of fibroblasts in culture, causing apoptosis (10) and regulates, in a negative manner, the replication of a DNA virus, the cytomegalovirus, which is closely dependent on the DNA-synthesis system of the host cell (11). Moreover, the present inventors have observed that the protein SSRP-1 is a nuclear phosphoprotein, the expression of which is closely correlated with the proliferative state of the cell, since it is high in the course of cell division and reduces dramatically when the cells start to differentiate. It has been demonstrated by confocal laser microscopy that there is an almost perfect coalignment between the nuclear location of SSRP1 and BrdU (5'-bromodeoxyuridine) in the S phase, and this has provided the first evidence of the fact that SSRP1 in eucaryotic cells is located in the region of the sites in which the genomic DNA replicates (12).

Altogether, these observations thus indicate a general correlation between SSRP-1 expression, DNA transcription, and cell growth and differentiation.

However, the specific biological role of SSRP proteins has not, up to now, been understood.

In order to fill this gap, the present inventors have produced monoclonal antibodies which react against the protein SSRP-1 and have used these antibodies to study the expression of this protein in various normal and abnormal tissues.

Monoclonal antibodies directed against the protein SSRP-1 have not been described in the prior art.

It has emerged from the studies carried out with the use of these antibodies that the level of expression of the protein SSRP-1 is not only correlated with the state of cell proliferation within a tissue but is altered in neoplastic tissues, when compared with the level of expression in the corresponding normal tissues.

The anti-SSRP-1 monoclonal antibodies of the present invention have therefore unexpectedly been found to be useful tools for the diagnosis and study of various types of neoplasia.

The subject of the present invention is N-Terminal fragment (from amino acid 60 to amino acid 467) of a monoclonal antibody which reacts against the protein SSRP-1 for the detection of neoplasias.

In the present description, this antibody will be referred to as an "anti-SSRP-1 monoclonal antibody" or sometimes, more simply, as an "anti-SSRP-1 antibody".

The term "protein SSRP-1" indicates the Structure-Specific Recognition Protein 1.

As will be described in further detail in the section relating to the examples, the anti-SSRP-1 monoclonal antibodies of the present invention were produced by the cell fusion technique, also known as somatic cell hybridization, which was described for the first time by Georges Kohler and Cesar Milstein in 1975.

The hybridoma cell lines producing anti-SSRP-1 antibodies were produced by fusing spleen cells producing antibodies with myeloma cells, with the use of polyethylene glycol as fusion agent.

These cells were obtained from mice immunized with the N-terminal fragment (amino-acids 60-467) of recombinant murine protein SSRP-1 (previously defined in the literature as T160, ref. 13), which is 98% identical to the human protein SSRP-1.

The amino-acid sequence SEQ ID NO:1 is the sequence of the N-terminal fragment of the murine protein SSRP-1 which was used as the immunogen.

The cells obtained as a result of the fusion were selected in HAT selective medium and the hybridomas were cloned and their ability to produce anti-SSRP-1 antibodies was checked. The check was carried out by ELISA (enzyme-linked immunosorbent assay) on the supernatant fluids of the hybridoma cells in culture.

Two of the positive clones were selected and were further cloned by the limit dilutions technique.

These hybridomas, referred to as R6G10 and R2H2, were deposited by the Applicant - in accordance with the Budapest Treaty on the international recognition of the deposit of micro-organisms for the purposes of patent proceedings - at the Interlab Cell Line Collection (ICLC), Centro di Biotecnologie Avanzate, L.go R. Benzi 10, 16132 Genoa, on 8th May 2001 (R6G10, entry number PD01003) and on 4th April 2001 (R2H2, entry number PD01002), respectively.

A hybridoma capable of producing monoclonal antibodies which react against the protein SSRP-1 therefore falls within the scope of the invention; this hybridoma is preferably selected from the hybridoma R6G10 (PD01003) and the hybridoma R2H2 (PD01002).

In order to produce monoclonal antibodies in sufficient quantities for use in the subsequent studies, the hybridomas R6G10 and R2H2 were grown as tumours in ascitic form in mice sensitized with pristane. Finally, the antibodies produced were purified from the ascitic fluids by precipitation with ammonium sulphate and protein-A affinity chromatography.

These antibodies represent a preferred embodiment of the anti-SSRP1 antibodies of the present invention; they will be referred to hereinafter as "R6G10 antibodies" and "R2H2 antibodies", respectively.

The R6G10 and R2H2 antibodies were characterized by the Ouchterlony isotype determination technique; the method used will be described in greater detail in the section relating to the examples.

It was clear from these characterization studies that the R6G10 and R2H2 antibodies belong to different IgG subclasses.

The R6G10 antibody in fact belongs to the IgG2a isotype, whereas the R2H2 antibody belongs to the IgG2b isotype.

In order to establish whether the various antibodies produced against the N-terminal of SSRP-1 recognize the same or different epitopes, N-terminal binding competition tests were performed with the use of radio-labelled antibodies.

This technique is referred to as "epitope mapping".

Briefly, the bases of polyvinyl chloride (PVC) micro-plate wells were covered with saturating quantities of antigens (N-terminal fragment of SSRP1, SEQ ID NO:1) and the R6G10 antibody or the R2H2 antibody, radio-labelled with I¹²⁵, was then added.

Increasing quantities (variable from 100 ng/ml to 10 microgrammes/ml) of the same antibody, not radio-labelled, or of other non-radio-labelled anti-SSRP-1 antibodies were then added as competitors for the N-terminal binding.

The amount of radioactivity (I¹²⁵) present in the base of the well was high if the two antibodies did not recognize the same epitope (and did not therefore compete with one another), whereas it was at low levels if the antibodies recognized the same epitope and the non-labelled antibody therefore displaced the labelled one.

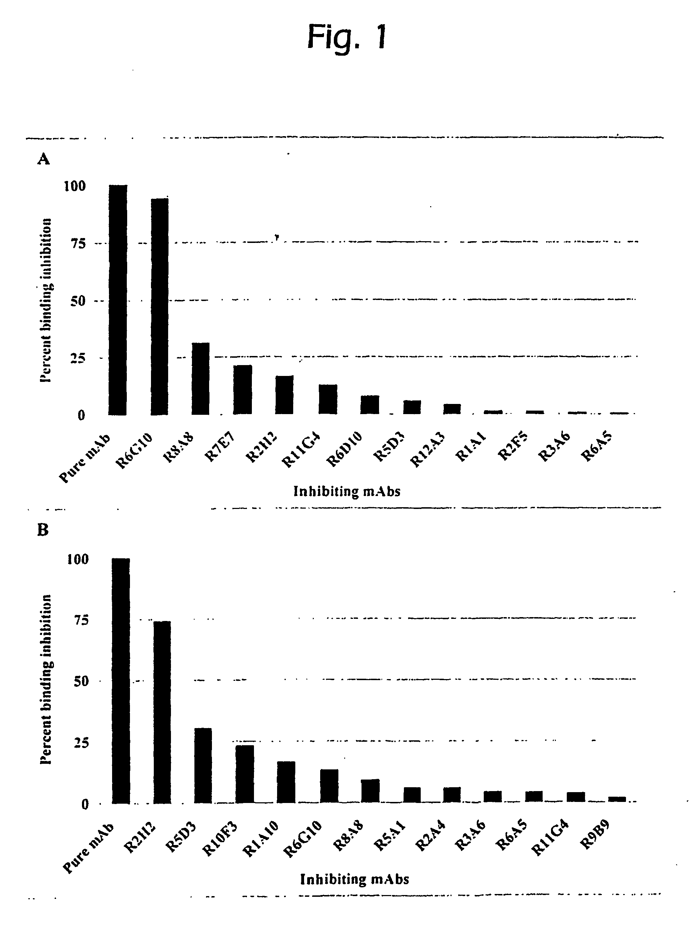

The results obtained (Figure 1) indicate clearly that, although both of the R6G10 and R2H2 antibodies are directed against the N-terminal of SSRP-1, they recognize different epitopes.

The reactivity profiles of the R6G10 and R2H2 antibodies were studied by the Western blotting technique on total protein extracts obtained from murine cell lines (NIH3T3) and human cell lines (HeLa).

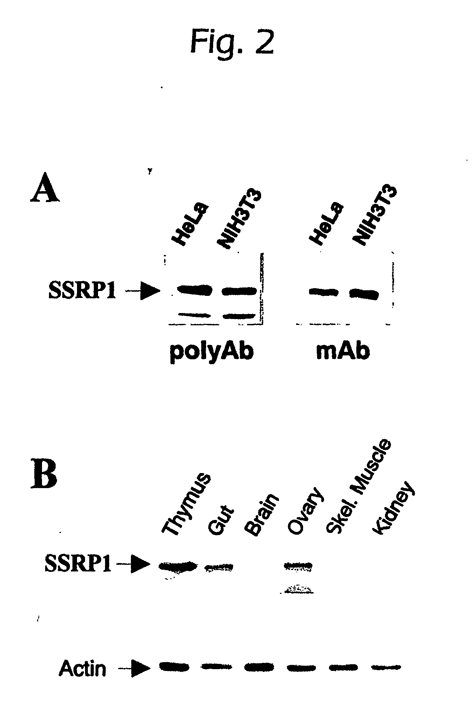

The proteins contained in the extracts were separated by electrophoresis and, with the use of the R6G10 antibody, a migrating band with an apparent molecular weight of 86 kDa, which corresponds to SSRP-1, was found (Figure 2, panel A).

This band is also recognized by rabbit polyclonal antibodies monospecific to the N-terminal of SSRP-1.

The same reactivity pattern was obtained for the R2H2 antibody (data not shown).

The R6G10 and R2H2 monoclonal antibodies were also used to analyze the expression of SSRP-1 in extracts from various murine tissues (Figure 2, panel B).

The thymus, the gut, and the ovary showed high levels of protein SSRP-1; the brain showed intermediate levels; the kidneys and the skeletal muscle showed zero levels.

With the use of these antibodies, it was thus possible to establish that there is a close correlation between the expression of the protein SSRP-1 and the percentage of proliferating cells in a given tissue.

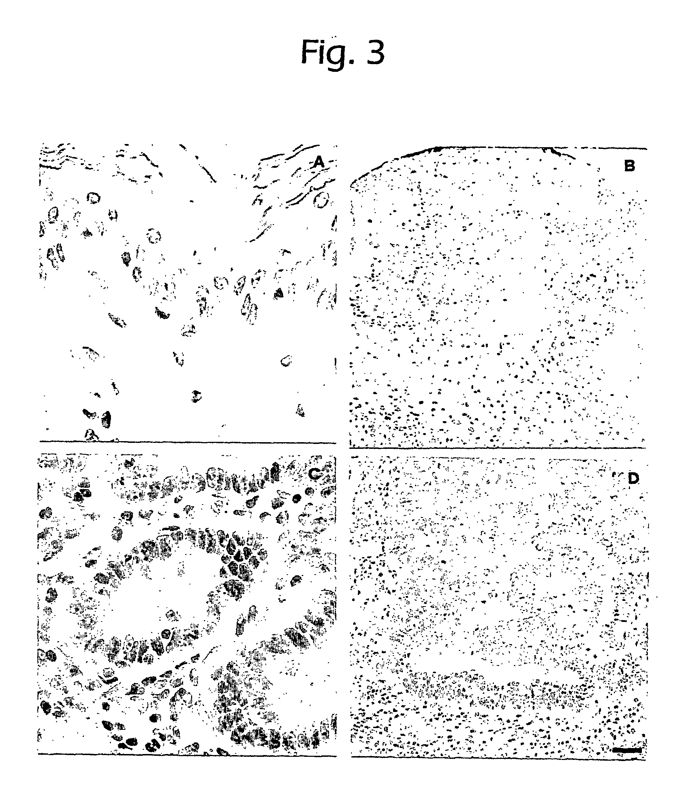

The expression of the protein SSRP-1 *in vivo* was then analyzed by immunohistochemical analysis of normal human tissue sections fixed in formalin and immersed in paraffin (Figures 3-5).

Consistently with what was established by Western blotting analysis, a strong coloration of nuclear SSRP-1 was found in tissues containing cells in course of proliferation.

In the epithelial tissue of normal skin (Figure 3A), the strongest reactivity was observed in the basal layer of the epithelium where DNA synthesis activity occurs. As the cells gradually migrate towards the upper layers and mature, the reactivity becomes weaker, which indicates a negative regulation of the expression of SSRP-1, in phase with cell maturation and the cessation of the DNA synthesis.

In colon mucosa sections, a similar profile was observed: an intense coloration at the base of the crypt, which decreases gradually as the cells gradually migrate towards the upper layers (Figure 3C).

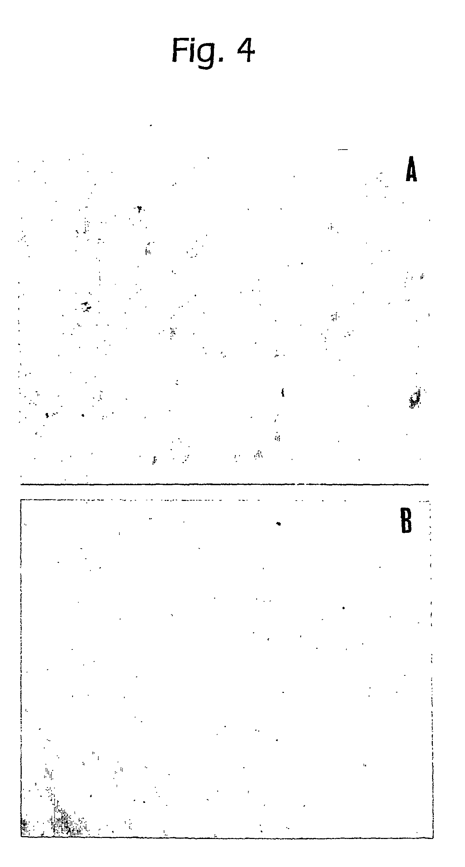

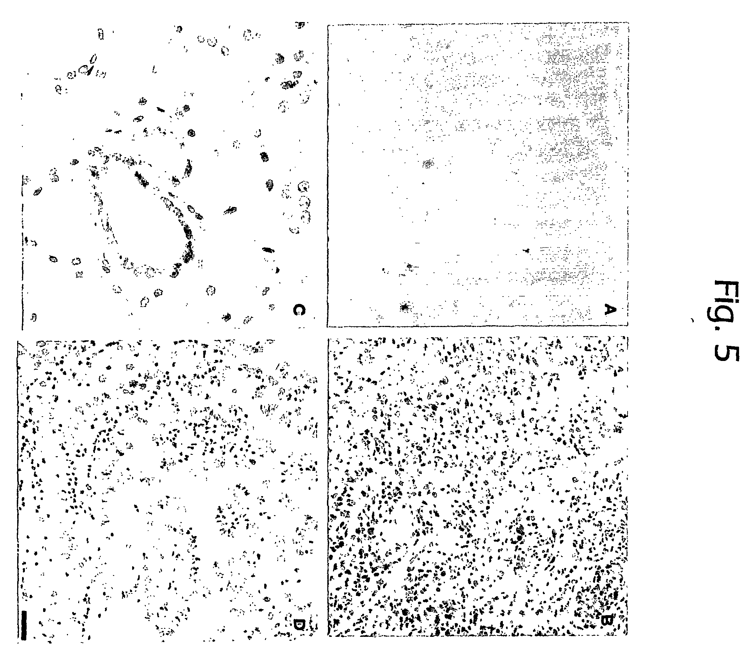

The normal human cerebral cortex sections showed a limited number of large neurones with nuclear positivity (Figure 4A). Moreover, in the internal granular layer of the folds of the cerebellum, no reaction was observed, whereas the Purkinje cells had both nuclear and cytoplasmic coloration (Figure 5A).

A completely different picture emerged, however, from the immunohistochemical coloration of neoplastic tissues.

Adenoma and adenocarcinoma of the large intestine showed a large number of positive SSRP-1 cells, whereas the residual areas of normal mucosa were less positive or negative (Figure 3D). In some adenocarcinoma samples, the positivity was both nuclear and cytoplasmic, particularly on the normal-tissue invasion front. Numerous lymphocytes infiltrating the tumorous mass were also coloured.

A considerable number of positive cells was also observed in malignant melanoma, in basal-cell carcinoma, and in squamous-cell carcinoma (Figure 3B); in some cases, the positivity extended to the cytoplasm of the neoplastic cells and to the lymphocytes of the stroma.

In medulloblastoma, more than 85% of the nuclei were positive.

In glial tumours, the results were less uniform: the positivity was less in tumours with a low degree of malignancy ("low grade") in comparison with the tumours with a high degree of malignancy ("high grade") (such as anaplastic astrocytoma and glioblastoma) (Figure 5B); in a single neoplasia, the large cells were more positive than the small cells.

The R6G10 and R2H2 antibodies were then reacted with sections from fully differentiated tissues such as, for example, skeletal muscle and kidney, in which the component of proliferating cells is only marginal. In these tests, the myotubes were negative, whereas the nuclei of the satellite cells were weakly positive (Figure 4B). Moreover, the protein SSRP-1 was not expressed by normal human kidney cells (Figures 5C), whereas an intense coloration was observable in the tissue sections derived from renal carcinoma (Figure 5D).

Comparison of the results obtained in normal cells and tumorous cells shows clearly and for the first time that the expression of the protein SSRP-1 is high and aberrant in tumour cells of some types.

The immunohistochemical analysis carried out on epithelial tissue and normal large intestine sections displayed a strong correlation between the expression of SSRP-1 and the programme of differentiation of the epithelial cells. The SSRP-1 coloration is much greater in the basal layers, whereas it decreases gradually as the cells gradually migrate towards the upper layers and differentiate. In contrast, a uniform strong positivity was observed in the neoplastic cells of the adenocarcinoma of the colon (particularly in the vicinity of the invasion front), and numerous positive cells were observed in skin tumours such as malignant melanoma, squamous-cell carcinoma, and basal-cell carcinoma. The expression profile was thus completely overturned in the neoplastic samples: the distribution of SSRP-1 was uniform throughout the tumorous tissue, the intensity of the coloration was significantly greater and, in some cases, the protein SSRP-1 was also present in the cytoplasm of the tumorous cells, unlike normal proliferating epithelial cells which show a precise nuclear location of this protein.

The coloration profile obtained in the cerebrum and cerebellum sections is particularly interesting since these tissues contain cells in which there is no detectable DNA synthesis but which are very active from the transcription point of view. The high levels of SSRP-1 observed in the protein extracts from these tissues can therefore be correlated with the synthesis of RNA and hence with transcription regulation activity. In the brain tissues, the changes in the expression of the protein SSRP-1 are even more dramatic. In normal cerebral cortex, only a few large neurones are positive, whereas the rest of the cortex is completely negative. In the tumorous mass, however, most of the cell nuclei become positive and the protein SSRP-1 also becomes detectable in the cytoplasm. Moreover, in the glial tissues, the positivity of the nuclei seems to correlate with the degree of malignancy since it is significantly less in tumours with low grade malignancy. With regard to the kidney and skeletal muscle, normal tissue sections appear negative with both of the antibodies, indicating that, in conditions of differentiation, the expression of SSRP1 is not detectable. The sections derived from renal carcinomas or fibrosarcomas, on the other hand, appear intensely coloured when reacted with one of the two antibodies, indicating that, as a result of neoplastic transformation, tissues which do not normally express SSRP-1, such as muscle and kidney, become intensely positive.

These results indicate clearly that the anti-SSRP-1 antibodies of the present invention are particularly useful as diagnostic tools, in particular, for the diagnosis of tumorous abnormalities.

The anti-SSRP1 monoclonal antibodies described above for diagnostic use, in particular for the diagnosis of tumours, therefore fall within the scope of the present invention. Particularly preferred for this purpose are the antibodies produced by the hybridomas R6G10 (PD01003) and R2H2 (PD01002).

As described above, the identification of tumorous cells and/or tissues is performed by studying the expression of the protein SSRP1 in the biological sample of interest. For this purpose, the anti-SSRP1 antibodies are used as specific reagents in immunoassays such as, for example, immunofluorescent, immunochemiluminescent, or immunohistochemical assays, or in any other technique based on antigen-antibody recognition and in which the antibody is labelled with a detectable molecule or with any other indicator means; these assays also include those in which the detection of the bond between protein SSRP1 (antigen) and anti-SSRP1 antibody is achieved with the use of a secondary antibody.

A method for the diagnosis, in vitro, of a tumorous abnormality, comprising the detection of the expression of the protein SSRP1 in a biological sample taken from a patient, the expression of the protein SSRP-1 being detected with the use of an anti-SSRP1 monoclonal antibody according to the present invention, therefore also falls within the scope of the present invention.

Amongst the tumorous abnormalities which can be detected by the method of the invention are, for example, tumours of the central nervous system, of the skin, of the muscle, and of the kidney.

The following examples are provided for illustrative purposes and are not intended to limit the scope of the present invention in any way.

### Example 1: production of anti-SSRP-1 monoclonal antibodies

A panel of monoclonal antibodies was produced with the use, as antigen, of recombinant murine protein SSRP-1, which is 98% identical to the human protein SSRP-1.

The N-terminal fragment of the murine protein SSRP-1 (amino-acids 60-467, SEQ ID NO:1), produced in E. coli as described by Gariglio et al. (9) and resuspended in PBS, was injected into the spleens of 10 anaesthetized BALB/c mice (200 µg/200 µl/mouse) through a direct opening in the peritoneum, in surgical conditions.

Boosters were administered (200 µg/200 µl/mouse) to these mice by intraperitoneal injection of the same antigen at intervals of about 15 days for 2 months.

Four days after the last injection, the spleen was removed for fusion with the myeloma standard cell line X63.Ag8.653 with the use of PEG 1500-1700 (Sigma, Germany) as fusion agent.

The hybrid cells obtained as a result of fusion were grown on RPMI-1640 medium (Gibco) containing HAT (hypoxanthine, aminopterin, thymidine) enriched with 5% horse serum (Hyclone) for the selection of the hybridomas.

### Example 2: selection of the hybridomas by ELISA assay

The ability of all of the hybridomas grown in selective medium to produce anti-SSRP-1 antibodies was then checked.

This check was carried out by ELISA ("enzyme-linked immunosorbent assay") on the supernatant liquids of the hybridoma cells in culture.

For this purpose, the same antigen was used as was used for the immunization and was immobilized on micro-titration plates with 96 wells (Nunc). For this purpose, the N-terminal fragment of the recombinant protein SSRP-1 (2 mg/ml) was absorbed on the bases of micro-titration plate wells in 0.004 M of a bicarbonate solution, pH 9.6, and incubated at 4°C for 18 hours. After the aspecific absorption sites had been blocked by means of a 2% bovine albumin solution (BSA) in double-distilled H₂O, the plates were washed with an 0.1 M solution of Tris-HCl, pH 7.5, containing 0.05% Tween 20 (Tris-Tween).

One hundred microlitres of supernatant fluid coming from the fusion cultures which were positive for growth of hybridomas were added to each well and the plates were incubated for 2 hours at 37°C.

The plates were then washed with Tris-Tween and 100 ml of polyvalent anti-mouse-Ig antibodies, marked with alkaline phosphatase (Pharmacia-Amersham), were added to each well for 2 hours at ambient temperature.

The plates were then washed 6 times with Tris-Tween and 100 ml of a solution containing the substrate (1 mg/ml p-nitrophenylphosphate in diethanolamine, pH 9.8) were then added.

Finally, the optical density (O.D.) of each well was measured at 405 nm. All of the hybridomas the supernatant fluid of which generated O.D. values greater than 2SD with respect to the cut-off obtained with supernatant fluids of cultures which were negative for growth of hybridomas (mean of 10 cultures negative for growth of hybridomas) were considered positive for the production of antibodies.

The supernatant fluids selected were derived from two hybridoma cultures which were thus designated R6G10 and R2H2 since they generated the highest O.D. values. These hybridomas were then recloned with 3 successive cycles by the limit dilution technique. This technique consists in the deposition of one hybridoma cell in each well of a plate with 96 wells, which are then assayed for their ability to produce anti-SSRP1 antibodies by the same technique as described above. Repeated cloning cycles permit stabilization of the hybridomas which thus retain their ability to produce antibodies over time.

### Example 3: isotype determination

The isotypes of the two antibodies R6G10 and R2H2 were determined by Ouchterlony radial immunodiffusion.

Briefly, this technique provides for radial diffusion of the antigen (that is, the antibody to be examined) in agarose gel with antibodies reactive against different isotypes of the same antigen.

To determine the isotypes of the murine R6G10 and R2H2 antibodies, sheep anti-mouse-antibody antibodies specific to the different isotypes of murine antibodies were used (commercial kit The Binding Site, Birmingham, UK, marketed in Italy by Bioline, Turin).

The sheep antibodies react with the murine antibodies and form a precipitation ring in the agarose gel, the diameter of which enables the concentration of the antigen used (mouse antibodies) and the type of antigen (isotype of the antibodies) to be established.

Micro-plates covered with agarose gel with sheep anti-mouse-Ig antibodies included were used for the procedure.

5 µl of supernatant fluid of the culture of the R6G10 hybridoma or of the R2H2 hybridoma were deposited in each well preformed in the gel. The supernatant fluids were concentrated 10 times beforehand by dialysis membrane on a sucrose layer.

After 72 hours, the diameter of the precipitation ring was assessed and it was thus possible to establish that the antibody R6G10 belonged to the IgG2a isotype and the antibody R2H2 to IgG2b.

### Example 4: Production of the monoclonal antibodies from tumours in ascitic form and their purification

In order to produce quantities of antibodies useful for their use, the hybridomas selected were grown as tumours in ascitic form in mice sensitized with pristane in order to induce a peritoneal ascitic effusion and to favour the production of antibodies. For this purpose BALB/c mice, 6 weeks old, were subjected to intraperitoneal inoculation with 0.3 ml of pristane. Five - seven days afterwards 1 x 10⁶ hybridoma cells were inoculated into the peritoneum. The ascitic liquid was then taken 15-18 days after inoculation and centrifuged to eliminate the cells, and the supernatant fluid was used for the purification of the antibodies.

The antibodies were then purified from the ascitic liquid by precipitation in ammonium sulphate (NH₄SO₄) (50% saturation) and affinity chromatography by Sepharose Protein A column (HiTrap, Amersham-Pharmacia Biotech).

Briefly, the antibodies of the two hybridomas, precipitated in NH₄SO₄, were dialyzed in a buffer containing 0.05 M Tris-HCl, 2M NaCl, pH 7.8. A volume of 5 ml containing a final concentration of 10 mg of antibody was then loaded into a Sepharose Prot-A column at a flow rate of 60 cm/hour. The column was then washed exhaustively until proteins disappeared from the washing liquid, which was monitored by UV spectrophotometer (280 nm). The IgGs retained by the Sepharose Protein A were then eluted with a 0.1 M citrate solution (pH 4.5 for IgG2a, pH 3.5 for IgG2b) and immediately buffered with a Tris-HCl solution (pH 8.8).

The monoclonal antibodies thus purified were then dialyzed against PBS at pH 7.4.

### Example 5: epitope mapping

To establish whether the antibodies produced against the N-terminal of SSRP-1 recognized the same or different epitopes, N-terminal binding competition tests ("epitope mapping") were performed with the use of antibodies radio-labelled with IODO-GEN (Pierce).

For this purpose, 10 mg of R6G10 or R2H2 monoclonal antibody (purified on protein A as described in Example 4) in 200 ml of PBS (pH 7.4) was transferred into test tubes covered with IODO-GEN (50 mg/ml) in which they were iodinated for 5 minutes at 4°C with I¹²⁵ (1 mCi/ml) (Amersham).

The reaction was then stopped by the addition of BSA (2% in PBS) and the iodized antibodies were separated by exclusion chromatography. The iodized antibodies (200 µl) were added to a column pre-packed with Sephadex G-10 (3 ml weight/vol. of resin) (PD10, Amersham-Pharmacia Biotech), previously washed with 10 ml of PBS, and were then separated from the free I¹²⁵ by exclusion chromatography on Sephadex G-10 resin. The fractions containing the labelled antibodies were detected by direct reading with a gamma-counter and the antibodies were kept at 4°C after the addition of glycerol (5%) to stabilize their activity. To establish whether the two antibodies recognized the same or different epitopes, the bases of PVC micro-plate wells were initially covered with saturating quantities of SSRP-1 (N-terminal) (2 µg/ml) with the use of the same conditions already described in Example 2 for the initial selection of the antibodies.

Firstly, the R6G10 or R2H2 antibodies, radio-labelled with I¹²⁵, were added (1 x 10⁵ cpm/well), and then quantities of the corresponding antibody (R6G10 or R2H2) or of other, non-radio-labelled antibodies, indicated in Figure 1, were added as N-terminal binding competitors, at concentrations capable of giving rise to 50% displacement of the radio-labelled antibody (250 ng/ml).

The quantity of radioactivity (I¹²⁵) present in the base of the well was high if the antibodies did not recognize the same epitope (and therefore did not compete with one another) and was at low levels if the antibodies recognized the same epitope and the non-labelled antibody thus displaced the labelled one.

The results obtained indicate clearly that R6G10 and R2H2 recognize different epitopes since they do not compete with one another for N-terminal binding of SSRP1 (Figure 1).

### Example 6: determination of the reactivity of R6G10 and R2H2 by Western blotting

The reactivity of the two R6G10 and R2H2 antibodies against the natural protein SSRP-1 was determined by the Western blotting technique.

Briefly, total protein extracts (30 µg) from human or murine cells (indicated in Figure 2) were separated on polyacrylamide gel in sodium dodecyl sulphate (SDS) (8% PAGE) by electrophoresis and were then transferred onto nitro-cellulose paper.

The R6G10 or R2H2 antibodies were then added, diluted 1:1000 in "blocking solution" (10 mM Tris-HCl, pH 7.5, 0.1 M NaCl, 0.1% Tween-20, 5% [weight/vol.] skimmed milk), and their binding to SSRP-1 was then displayed by rabbit anti-murine-Ig antibodies, conjugated with peroxidase.

Finally, the peroxidase was displayed by chemiluminescence reaction (Super Signal, Pierce). The results obtained show clearly that the R6G10 antibodies recognize, in the human and murine cell extracts, a single protein which migrates with an apparent molecular weight of about 86 kDa, similar to that expected for the protein SSRP1. Moreover, a similar band was recognized from a rabbit antiserum previously produced in the inventors' laboratory against the N-terminal fragment of SSRP1, confirming that the R6G10 antibodies react with the protein SSRP1 both of man and of mouse. Identical results in Western blotting were obtained with the R2H2 antibodies, showing that they also recognize the same protein SSRP-1.

### Example 7: investigations into the expression of the protein SSRP-1 in vivo

In order to check the tissue distribution *in vivo* of SSRP1 in normal or tumorous human tissues, the R6G10 and R2H2 antibodies were used in immunohistochemical analysis.

Tissue sections (5 microns) of biopsy samples were deparaffinated with xylene at ambient temperature for 5 minutes and then washed, first successively with solutions containing decreasing concentrations of ethanol in H₂O (99, 75, 50% ethanol) and, finally, with double distilled H₂O. The samples were then treated with H₂O₂ (3%) for 5 minutes, washed as described above, and then treated with citrate solution in a microwave oven. After washing 3 times with PBS, the sections were then reacted with the R6G10 and R2H2 antibodies for 60 minutes at ambient temperature (diluted 1:200 in PBS and 1% BSA). After washing with PBS, biotinylated rabbit anti-mouse-Ig antibodies were added to the sections for 15 minutes. The rabbit antibodies bound to the mouse antibodies were then incubated with a streptavidin/peroxidase complex (15 min.) and stained with a solution of DAB. Finally, the sections were washed, dehydrated, and incubated with permanent mounting liquid for microscope observation.

### Bibliographic references

1. Baxevanis A.D. and Landsmann D. 1995. Nucleic Acid Res. 23, 1604-13.
2. Bianchi M.E. 1995. The HMG-box domain. In: Lilley DMJ, ed. DNA-protein structural interactions, Oxford University Press, 177-200.
3. Bustin M., Lehen D.A. and Landsman D. 1990. Biochim. Biophys. Acta.1049, 231-43.
4. Spencer J.A., Barons M.H. and Olson E.N. 1999. J. Biol. Chem. 274, 15686-93.
5. Wittmeyer J. and Formosa T. 1997. Mol. Cel. Biol. 17, 4178-90.
6. Orphanides G., Wu W.-H., Lane W.S., Hampsey M. and Reinberg D. 1999. Nature 400, 284-8.
7. Dyer M.A., Hayes P.J. and Baron M.H. 1998. Mol. Cell. Biol. 5, 2617-28.
8. Xiang Y.-Y., Wang D.-Y., Tanaka M., Igarashi H., Naito Y., Ohtawara Y., Shen Q. and Sugimura H. 1996. Cancer Lett. 106, 271-8.
9. Gariglio M., Ying G.-G., Hertel L., Gaboli M., Clerc R.G. and Landolfo S. 1997. Exp. Cell Res. 236, 472-81.
10. Hertel L., Foresta P., Barbiero G., Ying G.-G., Bonelli G., Baccino F., Landolfo S. and Gariglio M. 1997. Biochimie 79, 717-23.
11. Gariglio M., Foresta P., Sacchi C., Lembo M., Hertel L. and Landolfo S. 1997. J. Gen. Virol. 78, 725-7.
12. Hertel L., De Andrea M., Bellomo G., Santoro P., Landolfo S. and Gariglio M. 1999. Exp. Cell Res. 250, 313-28.

## Claims

1. A monoclonal antibody capable of reacting with the N-terminal fragment from amino acid 60 to amino acid 467 of the high mobility group structure specific recognition protein 1 (SSRP-1), for the diagnosis of tumorous abnormalities.

2. The monoclonal antibody according to claim 1, which is obtainable from the hybridoma R6G10 (PD01003) or the hybridoma R2H2 (PD01002).

3. The monoclonal antibody according to Claim 1 or 2 for the diagnosis of tumorous abnormalities selected from the group consisting of tumours of the central nervous system, of the skin, of the muscle, and of the kidney.

4. A method for the in vitro diagnosis of a tumorous abnormality, comprising the detection of the expression of the high mobility group structure specific recognition protein 1 (SSRP-1) in a biological sample taken from a patient, the expression of the high mobility group structure specific recognition protein 1 (SSRP-1) being detected by a monoclonal antibody according to Claim 1.

## Patentansprüche

1. Monoklonaler Antikörper, der fähig ist, mit dem N-terminalen Fragment von der Aminosäure 60 zur Aminosäure 467 des strukturspezifischen High-Mobility-Group-Erkennungsproteins-1 (SSRP-1) zur Diagnose von tumorartigen Anomalien zu reagieren.

2. Monoklonaler Antikörper nach Anspruch 1, der von dem Hybridom R6G10 (PD01003) oder dem Hybridom R2H2 (PD01002) gewonnen werden kann.

3. Monoklonaler Antikörper nach Anspruch 1 oder 2 zur Diagnose von tumorartigen Anomalien, ausgewählt aus der Gruppe bestehend aus Tumoren des Zentralnervensystems, der Haut, des Muskels und der Niere.

4. Verfahren für die In-vitro-Diagnose einer tumorartigen Anomalität, umfassend die Feststellung der Expression des strukturspezifischen High-Mobility-Group-Erkennungsproteins-1 (SSRP-1) in einer von einem Patienten genommenen biologischen Probe, wobei die Expression des strukturspezifischen High-Mobility-Group-Erkennungsproteins-1 (SSRP-1) durch einen monoklonalen Antikörper gemäß Anspruch 1 festgestellt wird.

## Revendications

1. Anticorps monoclonal capable de réagir sur le fragment N-terminal de l'acide aminé 60 à l'acide aminé 467 de la protéine-1 de reconnaissance spécifique de structure (SSRP-1) du groupe de haute mobilité pour le diagnostic d'anomalies tumorales.

2. Anticorps monoclonal selon la revendication 1 que l'on peut obtenir à partir de l'hybridome R6G10(PD01003) ou de l'hybridome R2H2 (PD01002).

3. Anticorps monoclonal selon la revendication 1 ou 2 pour le diagnostic d'anomalies tumorales prises dans le groupe comprenant des tumeurs du système nerveux central, des tumeurs cutanées, musculaires et rénales.

4. Procédé pour le diagnostic in vitro d'une anomalie tumorale comprenant la détection de l'expression de la protéine-1 de reconnaissance spécifique de structure (SSRP-1) du groupe de haute mobilité dans un échantillon biologique prélevé du patient, l'expression de la protéine-1 de reconnaissance spécifique de structure (SSRP-1) du groupe de haute mobilité étant détectée par un anticorps monoclonal selon la revendication 1.
